# EUROPEAN PATENT SPECIFICATION

(11) **EP 3 126 327 B1**
(45) Date of publication and mention of the grant of the patent: **22.05.2019**
(21) Application number: 15715915.3
(22) Date of filing: 24.03.2015
(51) Int. Cl.: C07C 253/34, C07C 255/08, C01C 1/12, B01J 4/00, B01J 19/00, F28C 1/02, F28C 3/02, B01J 10/00, B01D 53/58, B01D 53/78

(54) **IMPROVED AMMONIA REMOVAL FROM REACTOR EFFLUENT**
VERBESSERTE AMMONIAKENTFERNUNG AUS REAKTORABWASSER
ÉLIMINATION AMÉLIORÉE D'AMMONIAC DE L'EFFLUENT D'UN RÉACTEUR

(30) Priority: 31.03.2014 CN 201410124761
(43) Date of publication of application: 08.02.2017
(73) Proprietor: Ineos Europe AG, 1180 Rolle (Vaud) (CH)
(72) Inventor: MCDONEL, Timothy Robert, Elburn, Illinois 60119 (US); COUCH, Jay Robert, Naperville, Illinois 60564 (US); WAGNER, David Rudolph, Naperville, Illinois 60564 (US); WACHTENDORF, Paul Trigg, Victoria, Texas 77904 (US)
(74) Representative: King, Alex
(86) International application number: PCT/US2015/022194
(87) International publication number: WO 2015/153190

(56) References cited:
- US-A1- 2001 006 614
- US-A1- 2009 173 234
- US-A1- 2010 233 057
- US-A1- 2012 157 740

## Description

### FIELD OF THE INVENTION

The disclosure is directed to an improved process and system for the manufacture of acrylonitrile or methacrylonitrile. In particular, the disclosure is directed to improved removal of ammonia from the effluent gas of an acrylonitrile reactor.

### BACKGROUND

Various processes and systems for the manufacture of acrylonitrile and methacrylonitrile are known; see for example, U.S. Patent Nos. 3,936,360; 3,433,822; 3,399,120; and 3,535,849. Propylene, ammonia, and oxygen (as an air component) are fed to an acrylonitrile reactor, which contains catalyst and operates as a fluidized bed. A conventional practice is to operate the reactor with an excess amount of ammonia in the feed with respect to the amount of propylene fed to the reactor. Some of the extra ammonia is burned in the reactor due to the extreme conditions before it can combine with propylene to form acrylonitrile. The remaining extra ammonia, commonly referred to as "excess ammonia," exits the reactor in the effluent gas. This gas then typically goes through a cooler and then to a quenching vessel to remove the excess ammonia. See e.g., U.S. Patent Nos. 3,936,360; 4,166,008, 4,334,965, 4,341,535, 5,895,635, and 6,793,776. US2009173234 describes (Fig. 11,1,2) a vessel (1) comprising an inlet (2) and a multi-level spray system (3) configured to receive a liquid (sprayed counter current to the gaseous stream), wherein the inlet (2) is located below the multi level spray system (3). The multi-level spray system includes a first and a second spray bar that extends substantially across the diameter of the vessel (1), each spray bar comprising a plurality of nozzles (5) of the hollow cone type configured to provide a spray of liquid, the spray nozzles (5) effective for providing a collective spray having a spray pattern that covers the surface area of the cross section of the vessel. Thus channelling of gas-to-be-treated about the walls (periphery) is inhibited.

Conventional processes and systems for removing excess ammonia have several common factors and one common issue. Conventional processes and systems typically attempt to remove the excess ammonia in the form of ammonium sulfate, and recover that ammonia sulfate at a minimum processing cost. The ammonium sulfate is a reaction product from the common use of sulfuric acid to quench the effluent stream from the acrylonitrile reactor to remove the excess ammonia. This leads to a common issue with conventional processes and systems described in the art. In order to minimize the cost of the ammonium sulfate recovery, there is an inherent loss in the production efficiency of the acrylonitrile process. The recovery of acrylonitrile products in the effluent stream and the removal of excess ammonia in an efficient manner present technical challenges not recognized by or solved in conventional processes and systems described in the art.

### SUMMARY

Accordingly, an aspect of this disclosure is to provide design criteria for the safe, effective and cost efficient removal of ammonia from the effluent gas of an acrylonitrile reactor. Specifically, this invention relates to the method and type of quench vessel used in the process to achieve the desired outcome.

In an aspect, an apparatus is provided for quenching of reactor effluent gas comprising acrylonitrile and ammonia. The apparatus comprises a quench vessel having a first portion and a second portion, the first portion located below the second portion. The first portion of the quench vessel comprises an inlet configured to receive a gas stream, the gas stream comprising acrylonitrile and ammonia. The second portion of the quench vessel comprises a multi-level spray system that is configured to receive a quench liquid, wherein the quench liquid comprises an acid. The multi-level spray system comprises at least a first spray bar and a second spray bar, each bar extending substantially across a diameter of a quench vessel. Each spray bar is configured to receive the quench liquid. The first spray bar is located below and parallel to the second spray bar. Each bar comprises arms that extend substantially perpendicular to its respective bar, each arm having at least two extenders extending substantially perpendicular to its respective arm. Each extender comprises a nozzle configured to downwardly spray a hollow cone spray of the quench liquid. Spray from the first and second bars, in combination, provides a downward wall of projected spray that covers more surface area of a cross section of the quench vessel parallel to the diameter of the vessel than the surface area covered by the first projected spray alone.

The nozzles of the first spray bar are spaced so that a portion of each hollow cone spray of quench liquid from each nozzle of the first spray bar overlaps with a portion of another hollow cone spray of quench liquid from at least one adjacent nozzle to provide a plurality of first overlaps of the quench liquid, wherein each first overlap has a first overlap center.

The nozzles of the second spray bar are spaced so that a portion of each hollow cone spray of quench liquid from each nozzle of the second spray bar overlaps with a portion of another hollow cone spray of quench liquid from at least one adjacent nozzle to provide a plurality of second overlaps of the quench liquid, wherein each second overlap has a second overlap center. The nozzles of the second spray bar are spaced so that the center of at least one hollow cone spray of the second spray bar is vertically offset from the centers of the hollow cone sprays of the first spray bar, and the center of at least one hollow cone spray of the second spray bar is vertically aligned with at least one first overlap center of the first spray bar.

The above and other aspects, features and advantages of the present disclosure will be apparent from the following detailed description of the illustrated embodiments thereof which are to be read in connection with the accompanying drawings.

### BRIEF DESCRIPTION OF THE DRAWINGS

A more complete understanding of the exemplary embodiments of the present invention and the advantages thereof may be acquired by referring to the following description in consideration of the accompanying drawings, in which like reference numbers indicate like features and wherein:
FIG. 1 is a side view of a quench vessel in accordance with at least one aspect of the disclosure;
FIG. 2 is a bottom view of first spray bar of a quench vessel taken along line 2-2 in FIG. 1 in accordance with at least one aspect of the disclosure; and
FIG. 3 is a bottom view of a second spray bar of a quench vessel taken along line 3-3 in FIG. 1 in accordance with at least one aspect of the disclosure.
FIG. 4 illustrates a flow diagram in accordance with aspects of the disclosure.

### DETAILED DESCRIPTION

In an aspect, an apparatus is provided for quenching of reactor effluent gas comprising acrylonitrile and ammonia. The apparatus may be suitable as a single stage quench apparatus, meaning that the quenching of reactor effluent gas, wherein excess ammonia is removed from the reactor effluent gas, is accomplished in a single quench vessel. The apparatus comprises a quench vessel having a first portion and a second portion, the first portion located below the second portion. The first portion of the quench vessel comprises an inlet configured to receive the reactor effluent gas stream, the gas stream comprising acrylonitrile and ammonia. The second portion of the quench vessel comprises a multi-level spray system that is configured to receive a quench liquid, wherein the quench liquid comprises an acid. The multi-level spray system comprises at least a first spray bar and a second spray bar, each bar extending substantially across a diameter of a quench vessel. The first spray bar is located below and parallel to the second spray bar. Each bar comprises arms that extend substantially perpendicular to its respective bar, each arm having at least two extenders extending substantially perpendicular to its respective arm. Each extender comprises a nozzle configured to downwardly spray a hollow cone spray of the quench liquid. Spray from the first and second bars, in combination, provides a downward wall of projected spray that covers more surface area of a cross section of the quench vessel parallel to the diameter of the vessel than the surface area covered by the first projected spray alone. In an aspect, each hollow cone spray defines a center or center line below its respective nozzle and equidistant from the walls of the hollow cone spray.

In one aspect, the multi-level spray system extends across about 85% or more of the diameter of the quench vessel, in another aspect, about 90% or more, in another aspect, about 95% or more, and in another aspect, about 99% or more. In a related aspect, the multi-level spray system is effective for providing a spray pattern that covers about 85% or more of the diameter of the quench vessel, in another aspect, about 90% or more, in another aspect, about 95% or more, and in another aspect, about 99% or more.

In an aspect, each extender has a downward facing nozzle at a distal end of the extender.

The nozzles of the first spray bar are spaced so that a portion of each hollow cone spray of quench liquid from each nozzle of the first spray bar overlaps with a portion of another hollow cone spray of quench liquid from at least one adjacent nozzle to provide a plurality of first overlaps of the quench liquid, wherein each first overlap has a first overlap center. The plurality of first overlaps and non-overlapping portions of each hollow cone spray of quench liquid from the nozzles of the first spray bar collectively comprise a first projected spray of quench liquid from the first spray bar.

The nozzles of the second spray bar are spaced so that a portion of each hollow cone spray of quench liquid from each nozzle of the second spray bar overlaps with a portion of another hollow cone spray of quench liquid from at least one adjacent nozzle to provide a plurality of second overlaps of the quench liquid, wherein each second overlap has a second overlap center. The plurality of second overlaps and non-overlapping portions of each hollow cone spray from the nozzles of the second spray bar collectively form a second projected spray from the second spray bar.

The nozzles of the second spray bar are spaced so that the center of at least one hollow cone spray of the second spray bar is vertically offset from the centers of the hollow cone sprays of the first spray bar, and the center of at least one hollow cone spray of the second spray bar is vertically aligned with at least one first overlap center of the first spray bar. The first projected spray and the second projected spray, in combination, may provide a downward wall of spray that covers more of a surface area of a horizontal cross section of the quench vessel below the first spray bar than a surface area of the same horizontal cross section covered by the first projected spray alone. In an aspect, the second projected spray fills in a plurality of gaps between the hollow cone spray of quench liquid from the nozzles of the first spray bar.

An object of this disclosure is to provide design criteria for the safe, effective and cost efficient removal of ammonia from the effluent gas of an acrylonitrile reactor. Specifically, this disclosure relates to the method and type of quench vessel used in the process to achieve the desired outcome. It has been found that the following design features result in excellent quench performance with regard to both the removal of the excess ammonia and the recovery of the acrylonitrile reaction products.

Those skilled in the art will recognize that in accordance with the disclosure, the apparatuses and methods disclosed herein may be used for effective neutralization and/or quenching of a wide variety of process gas streams. A preferred process gas stream is an acrylonitrile reactor effluent. Regardless of the temperature of the quench operation, which may range from an outlet temperature of about 32.2°C to 93.3°C (90°F to 200°F), the spray quench design of multiple layers of overlapping hollow cone sprays consistently removes high levels of excess ammonia from the reactor effluent products.

To accommodate the multiple layer spray quench, a series of spray bars may be added to a quenching vessel of appropriate size for the desired linear velocity of reactor effluent products in an upward direction. The spray bars may comprise circular bars, e.g., circular pipes. The spray bars may face downward such that gravity will drain the liquid from the headers or bars and sprays when not in use.

Those skilled in the art will recognize that in accordance with the disclosure, the number of spray bars may be varied to achieve desired quenching of reactor effluent products. For example, the number of spray bars may vary from 4 to 10. Those skilled in the art will recognize that in accordance with the disclosure, the distance between spray bars may be varied to achieve desired quenching of reactor effluent products. For example, the spray bars may have a separation of about 0.65 meters to about 1.4 meters, in another aspect, about 0.75 to about 1.3 meters, in another aspect, about 0.85 to about 1.2, and in another aspect, about 0.95 to about 1.1.

Circulation rates for the liquid quenching fluid that contains the sulfuric acid to remove the excess ammonia may be about 12:1 to 25:1 based on the ratio of recycle sulfuric acid : fresh sulfuric acid to allow for sufficient mass flux through the apparatus, thereby providing good liquid distribution and vapor/liquid contacting of the vapor with the liquid in the quench vessel or tower for effective ammonia removal. In an aspect, this circulation rate may be about 18:1.

Hollow cone spray nozzles with ramp entry and no internal parts may be used to reduce or prevent plugging by any circulating solids that might accumulate in the quenching stream.

Individual block valves and flow meters may be used on each of the spray bars to allow for adjustment and verification of flow.

Sulfuric acid may be added to the recycle quench liquid flow that is recycled to provide a sufficient amount of acid to maintain a constant quenching stream pH between about 3.0 and 6.0, in another aspect, about 4.5 and 6, with 5.5 being a preferred pH.

Ammonium Sulfate concentrations in the circulating stream may be maintained at a level from about 3% to 20% by weight, depending on the temperature of operation of the quench vessel. Operation at the higher end of the range of ammonium sulfate concentrations may require addition of a clean stream of water or recirculation of a clean stream of water from the purification and recovery operations in the manufacturing process for acrylonitrile to maintain a suitable level of ammonium sulfate within the desired range.

Organic removal from the quenching stream may be accomplished by conventional methods using a stripper or waste water column to remove the desired organics from the stream via an overhead vapor stream that is circulated back to the quench vessel and inserted back in to the vapor stream of reactor effluent beneath the sprays in the vessel.

The cone of quench liquid being sprayed from the hollow cone sprays may have an angle of about 70 to 100 degrees. The hollow cone sprays may be spaced evenly throughout the vessel on a level plane such that there is approximately a 50% overlap of the projected spray pattern of the 70 to 100 degree spray outlet from the cones. Subsequent layers of spray bars may be spaced such that the center of the cones below each layer is located at the center of the overlap from the layer above. Once a two layer pattern is established where there this sufficient coverage from the walls of the vessel to the center of the vessel, the pattern may be repeated in each additional pair of layers. The vertical alignment of nozzles and overlap centers as described with respect to various embodiments herein can apply to two immediately adjacent layers of spray bars or to two spray bars having intervening layers of spray bars (e.g., alternating layers of spray bars).

Nominal 2.54 cm (1 inch) inlet spray cone nozzles may be used. The nozzles may spray quench liquid through an outlet that is facing downward and may be perpendicular to the spray bars. This configuration may be used to facilitate easy installation and removal of nozzles, as well as complete draining when not in use.

In some instances, the loss in recovery efficiency in conventional processes and systems can be in excess of 10% higher than a plant with the quenching system described in this disclosure. Accordingly, for a given, desired degree of ammonia recovery from the reactor effluent, *e.g*., 95% recovery or 99% recovery, the overall height of the quench vessel, or height required to achieve each theoretical vapor-liquid equilibrium contacting stage in attaining the desired recovery, may be reduced in excess of 10% relative to such conventional processes, resulting in reduced material and operating costs.

It is desirable that the method used to quench the effluent gases from the acrylonitrile reaction in order to remove the excess ammonia is done so as efficiently as possible in regards to both the removal of the ammonia and the recovery of the acrylonitrile products in the effluent stream.

The process of the present disclosure will now be described in further detail with reference to the figures.

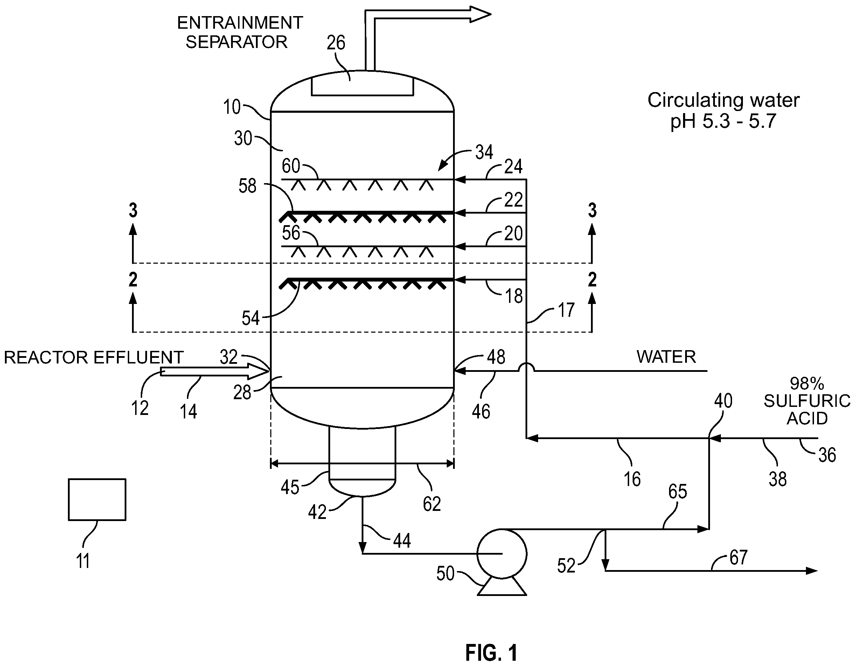

FIG. 1 is a side view of a quench vessel 10 in accordance with at least one aspect of the disclosure. Quench vessel 10 is configured to quench reactor effluent 12. Reactor effluent 12 may be obtained by the direct reaction of propane or propylene, ammonia and oxygen containing gas in a reaction zone (not shown) in the presence of a catalyst. Reactor effluent 12 is transported to quench vessel 10 via conduit 14, wherein the hot reactor effluent gases are cooled by contact with an aqueous stream or quench liquid 16 entering quench vessel 10 via lines 18, 20, 22, and 24. The cooled effluent gas comprising acrylonitrile (including coproducts such as acetonitrile, hydrogen cyanide and impurities) may then be passed through an entrainment separator 26, and then to an absorber column (not shown).

As shown in FIG. 1, quench vessel 10 comprises a first portion 28 and a second portion 30, wherein first portion 28 is located below the second portion 30. First portion 28 of the quench vessel 10 comprises an inlet 32 configured to receive a gas stream or reactor effluent 12, wherein the gas stream or reactor effluent 12 comprises acrylonitrile and ammonia. Second portion 30 of the quench vessel 10 comprises a multi-level spray system 34 that is configured to receive an aqueous stream or quench liquid 16, wherein the aqueous stream or quench liquid 16 comprises an acid 36. Acid 36 may be added via line 38 to quench liquid 16 at juncture 40. Acid 36 may be any suitable acid, e.g., sulfuric acid (such as 98 wt-% sulfuric acid). Quench liquid 16 comprises liquid effluent exiting bottom 42 of quench vessel 10 and through line 44. Water may be added via line 46 to quench vessel 10 through inlet 48, or otherwise may be added to quench liquid 16 or elsewhere in the liquid recycle loop formed by streams 17, 44, and 65. Quench liquid 16 is circulated through line 44 and back to lines 18, 20, 22, and 24, using pump 50. A stream 67 may be withdrawn as part of the liquid effluent exiting through line 44, in order to maintain a relatively constant mass flow in the liquid recycle loop by offsetting the liquid added via lines 38 and 46. Stream 67 removes formed neutralization reaction products (*e*.*g*., ammonium sulfate) and is also useful for preventing the accumulation of unwanted products in the liquid recycle loop, such as corrosion products. Effluent exiting bottom 42 of quench vessel 10 may be drawn from line 44 at siphon point 52.

Multi-level spray system 34 comprises at least a first spray bar 54, corresponding to line 18, and a second spray bar 56 corresponding to line 20. As shown in FIG. 1, multi-level spray system 34 also comprises spray bar 58, corresponding to line 22, and spray bar 56, corresponding to line 24. Spray bars 54, 56, 58, and 60 extend substantially across a diameter 62 of quench vessel 10. As shown, spray bar 54 is located below spray bar 56, and substantially parallel to spray bar 56. Spray bar 58 is located above spray bar 56, and below spray bar 60. Spray bar 58 is substantially parallel to spray bar 60.

In an aspect, controller 11 may be configured to process one or more signals corresponding to a measured parameter, e.g., the temperature measured by a temperature controller (not shown in FIG. 1). Controller 11 may be configured to determine whether the measured parameter is above or below a predetermined parameter range. Those skilled in the art will recognize that in accordance with the disclosure, the measured parameter may any suitable parameter useful in operation of the quench vessel, e.g., a temperature measured by the temperature controller at a predetermined location, or a liquid level measured by a level controller (not shown in FIG. 1) in boot 45 of the quench vessel 10, or a flow controller (not shown in FIG. 1). Controller 11 may be configured to adjust operation of one or more devices via communication lines or wireless communications (not shown in FIG. 1) if the measured parameter is below or above a predetermined parameter range. For example, controller 11 may be configured to adjust the amount of a stream conveyed to quench vessel 10, e.g., streams such as reactor effluent 12, water (conveyed through line 46 to quench vessel 10), and/or quench liquid 16 (including acid 36 conveyed through line 38). Those skilled in the art will recognize that in accordance with the disclosure, controller 11 may be configured to control operation of pump 50 and/or operation of other pumps and/or valves associated with the above streams in order to meet the predetermined range. Those skilled in the art will recognize that controller 11 or a similar controller may be located remote from a temperature controller, a level controller, or flow controller (not shown in FIG. 1), or may be located at and comprise a temperature controller, a level controller, or a flow controller.

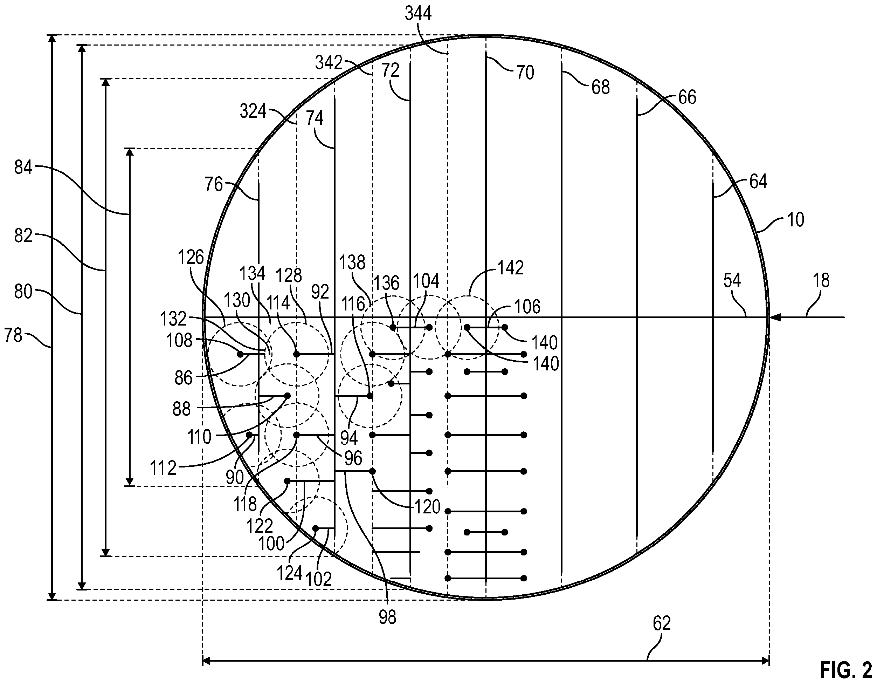

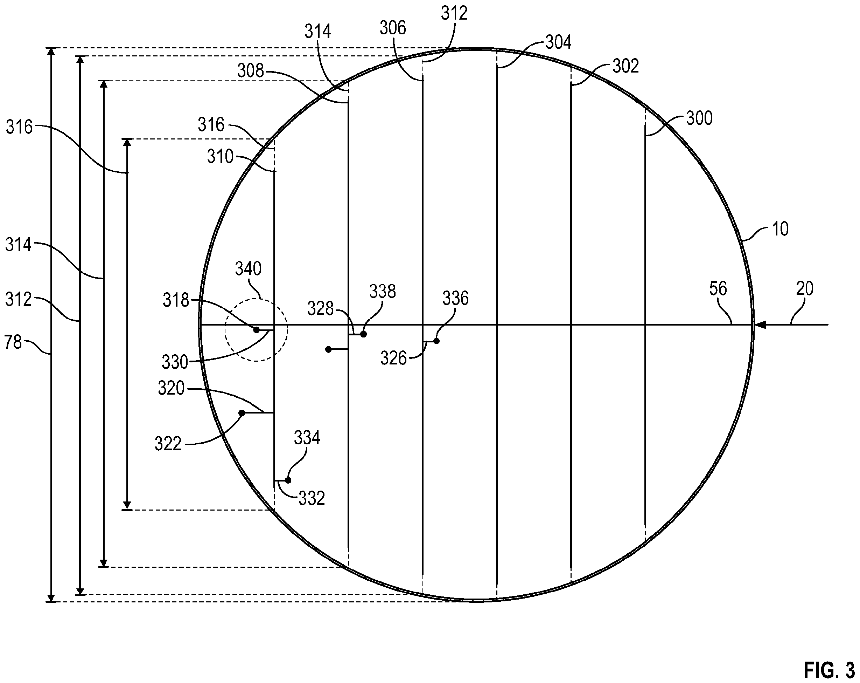

FIG. 2 is bottom view of spray bar 54 in quench vessel 10 taken along line 2-2 in FIG. 1. FIG. 3 is a bottom view of spray bar 56 in quench vessel 10 taken along line 3-3 in FIG. 1. Spray bar 58 may have a configuration similar to spray bar 54. Spray bar 60 may have a configuration similar to spray bar 56. Each spray bar may comprise a series of spray arms that extend substantially perpendicular to their respective spray bar. As used herein, "substantially perpendicular" means an angle that is about ± 15° to perpendicular, in another aspect, about ± 10°, and in another aspect, about ± 5°. The following discussion focuses on the lower left quadrant of spray bar 54 shown in FIG. 2. Those skilled in the art will recognize that similar structure, such as mirror structure, may be provided throughout the entirety of spray bar 54. As shown in FIG. 2, spray bar 54 comprises a series of spray arms 64, 66, 68, 70, 72, 74, and 76. Spray arm 70 extends substantially across diameter 78 of quench vessel 10. Diameter 78 is perpendicular to diameter 62. Spray arms 64, 66, 68, 72, 74, and 76 extend substantially across a respective chord of quench vessel 10, wherein each chord is substantially parallel to diameter 78. As shown in FIG. 2, spray arm 72 extends substantially across respective chord 80, spray arm 74 extends substantially across respective chord 82, and spray arm 76 extends substantially across respective chord 84.

Each spray arm of each spray bar may have two or more extenders. As shown in FIG. 2, spray arm 76 has extenders 86, 88, and 90. Spray arm 74 has extenders 92, 94, 96, 98, 100, and 102. Spray arm 72 has extenders 104, and spray arm 70 has extenders 106. Each extender extends substantially perpendicular to its respective spray arm. Each extender comprises a spray nozzle at an end of its respective extender, wherein each spray nozzle faces downward. In an aspect, each nozzle is configured to downwardly spray a hollow cone spray of the quench liquid, wherein each hollow cone spray defines a center equidistant from the walls of the hollow cone spray. As shown in FIG. 2, extender 86 has nozzle 108, extender 88 has nozzle 110, and extender 90 has nozzle 112. As shown in FIG. 2, extender 92 has nozzle 114, extender 94 has nozzle 116, extender 96 has nozzle 118, extender 98 has nozzle 120, extender 100 has nozzle 122, and extender 102 has nozzle 124.

In an aspect, the nozzles of the first spray bar may be spaced so that a portion of a first hollow cone spray of quench liquid from a first nozzle of the first spray bar overlaps with a portion of a second hollow cone spray of quench liquid from a second nozzle of the first spray bar to provide a first overlap of the quench liquid, the first overlap having a first overlap center. For example, as shown in FIG. 2, a portion of hollow cone spray 126 of nozzle 108 overlaps with a portion of hollow cone spray 128 of nozzle 114 to provide a first overlap 130 having an overlap center 132. As shown in FIG. 2, gaps 134 are defined where there is no overlap between hollow cone sprays of adjacent nozzles of spray bar 54. Extenders 104 of spray arm 72 comprise nozzles 136. Nozzles 136 are configured to produce hollow cone sprays 138. Extenders 106 of spray arm 70 comprise nozzles 140. Nozzles 140 are configured to produce hollow cone sprays 142.

In an aspect, each hollow cone spray of the first spray bar may have the same diameter of at least one other hollow cone spray of the first spray bar. Each hollow cone spray of the second spray bar may have the same diameter of at least one other hollow cone spray of the second spray bar. Each hollow cone spray of the first spray bar may have the same diameter of at least one hollow cone spray of the second spray bar.

In an embodiment, along the axis defined by spray arm 70, spray arm 68 is the mirror of spray arm 72, spray arm 66 is the mirror of spray arm 74, and spray arm 64 is the mirror of spray arm 76. Those skilled in the art will recognize that in accordance with the disclosure, similar structure may be provided in the upper quadrants, i.e., the upper quadrants are a mirror image of the lower quadrants shown in FIG. 2, with the mirror image of extenders, nozzles, hollow cone sprays, overlaps, overlap centers, and gaps.

In an aspect the number of nozzles on spray arm 76 may be about six (i.e., three nozzles in each of the two quadrants through which spray arm 76 extends). In an aspect the number of nozzles on spray arm 74 may be about ten to fourteen (five to seven in each of the two quadrants through which spray arm 74 extends). In an aspect the number of nozzles on spray arm 72 may be about thirty to thirty-six (i.e., fifteen to eighteen nozzles in each of the two quadrants through which spray arm 74 extends). In an aspect the number of nozzles on spray arm 70 may be about thirty-eight to forty (i.e., nineteen to twenty nozzles in each of the two half circles through which spray arm 70 extends).

FIG. 3 is a bottom view of spray bar 56 shown in FIG. 1. Spray bar 60 may have a configuration similar to spray bar 56. The following discussion focuses on the lower left quadrant of spray bar 56 shown in FIG. 3. Those skilled in the art will recognize that similar structure, such as mirror structure, may be provided throughout the entirety of spray bar 56. As shown in FIG. 3, spray bar 56 comprises a series of spray arms 300, 302, 304, 306, 308, and 310. Spray arms 300, 302, 304, 306, 308, and 310 extend substantially across a respective chord of quench vessel 10, wherein each chord is substantially parallel to diameter 78. As shown in FIG. 3, spray arm 306 extends substantially across respective chord 312, spray arm 308 extends substantially across respective chord 314, and spray arm 310 extends substantially across respective chord 316.

As previously noted, each spray arm of each spray bar may have two or more extenders. As shown in FIG. 3, spray arm 310 has extenders 318, 320, and 322. Spray arm 308 has extenders 328, and spray arm 306 has extenders 326. Each extender extends substantially perpendicular to its respective spray arm. Each extender comprises a spray nozzle at an end of its respective extender, wherein each spray nozzle faces downward. In an aspect, each nozzle is configured to downwardly spray a hollow cone spray of the quench liquid, wherein each hollow cone spray defines a center equidistant from the walls of the hollow cone spray. As shown in FIG. 3, extender 318 has nozzle 330, extender 320 has nozzle 332, and extender 322 has nozzle 334. As shown in FIG. 3, extenders 328 have nozzles 338, and extenders 326 have nozzles 336. Nozzle 330 is configured to produce a hollow cone spray 340 of quench liquid. Hollow cone spray 340 overlaps with gap 134 shown in FIG. 2. In an embodiment, the center of hollow cone spray 340 is directly over overlap center 132.

Spray arm 310 is located on chord 316. Chord 316 is located directly above the chord 324 shown in FIG. 2. Chord 324 is between spray arms 76 and 74. In an embodiment chord 324 is equidistant between spray arms 76 and 74. Chord 308 is located directly above chord 342 shown in FIG. 2. Chord 342 is between spray arms 74 and 72. In an embodiment chord 342 is equidistant between spray arms 74 and 72. Chord 306 is located directly over chord 344 shown in FIG. 2. Chord 344 is between spray arms 72 and 70. In an embodiment chord 344 is equidistant between spary arms 74 and 72. In an embodiment, spray arm 300 is the mirror of spray arm 310, spray arm 302 is the mirror of spray arm 308, and spray arm 304 is the mirror of spray arm 306.

In an aspect, centers of the hollow cone sprays of nozzles of spray bar 56 are located over, i.e., vertically aligned with, respective overlap centers of spray bar 54, wherein spray of quench liquid from spray bar 56 and spray of quench liquid from the spray bar 54 covers more of a surface area of a horizontal cross section of the quench vessel 10 that is below spray bar 54 than a surface area of the same horizontal cross section covered by the spray of quench liquid from spray bar 54 alone. Similarly, in an aspect, centers of the hollow cone sprays of nozzles of spray bar 60 are located over respective overlap centers of spray bar 58, wherein spray of quench liquid from spray bar 60 and spray of quench liquid from the spray bar 58 covers more of a surface area of a horizontal cross section of the quench vessel 10 that is below spray bar 58 than a surface area of the same horizontal cross section covered by the spray of quench liquid from spray bar 58 alone.

In an aspect, the nozzles of spray bar 54 may be spaced so that a portion of each hollow cone spray of quench liquid from each nozzle of spray bar 54 overlaps with a portion of another hollow cone spray of quench liquid from at least one adjacent nozzle to provide a plurality of first overlaps of the quench liquid, wherein each first overlap has a first overlap center. The plurality of first overlaps and non-overlapping portions of each hollow cone spray of quench liquid from the nozzles of spray bar 54 collectively comprise a first projected spray of quench liquid from spray bar 54.

In an aspect, the nozzles of spray bar 56 may be spaced so that a portion of each hollow cone spray of quench liquid from each nozzle of spray bar overlaps 56 with a portion of another hollow cone spray of quench liquid from at least one adjacent nozzle to provide a plurality of second overlaps of the quench liquid, wherein each second overlap has a second overlap center. The plurality of second overlaps and non-overlapping portions of each hollow cone spray from the nozzles of spray bar 56 collectively form a second projected spray from spray bar 56.

In an aspect, the nozzles of spray bar 56 may be spaced so that the center of at least one hollow cone spray of spray bar 56 is vertically offset from the centers of the hollow cone sprays of spray bar 54. In an embodiment, the center of at least one hollow cone spray of spray bar 56 is centered over at least one first overlap center of spray bar 54. The first projected spray and the second projected spray, in combination, may provide a downward wall of spray that covers more of a surface area of a horizontal cross section of quench vessel 10 that is below spray bar 54 than a surface area of the same cross section covered by the first projected spray alone. In an aspect, the second projected spray fills in a plurality of gaps 134 between the hollow cone spray of quench liquid from spray bar 54.

In an embodiment, there may be four to ten spray bars in quench vessel 10. Thus, there may be two to five pairs of first and second spray bars.

Those skilled in the art will recognize that in accordance with the disclosure, the apparatus described above may be used in a method for quenching and neutralizing a reactor effluent gas comprising an acidic or basic gas component. The method may comprise introducing the reactor effluent gas into a bottom portion of the apparatus, and introducing quench liquid through the first and second plurality of nozzles, whereby the quench liquid contacts the reactor effluent gas in a counter-current manner to neutralize and quench the reactor effluent gas.

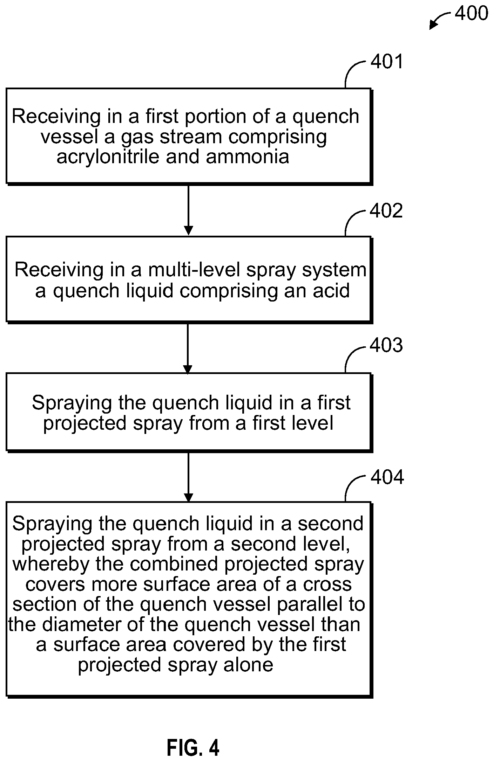

FIG. 4 illustrates a flow diagram of a method 400 in accordance with aspects of the disclosure. Method 400 may be carried out using apparatus previously described. Step 401 comprises receiving in a first portion of a quench vessel a gas stream comprising acrylonitrile and ammonia. Step 402 comprises receiving in a multi-level spray system a quench liquid comprising an acid, the multi-level spray system located in a second portion of the quench vessel, the second portion located above the first portion. The multi-level spray system may comprise at least a first spray bar and a second spray bar, wherein the first spray bar and the second spray bar extend substantially across a diameter of the quench vessel, wherein the first spray bar is located below the second spray bar, and substantially parallel to the second spray bar. The method may comprise spraying the quench liquid from the first and second spray bars. Each spray bar may comprise a plurality of spray arms that extend substantially perpendicular to their respective spray bar, wherein at least one spray arm of the first spray bar, and at least one spray arm of the second spray bar has two or more extenders. Each extender may extend substantially perpendicular to its respective spray arm, each extender comprising a spray nozzle, the spray nozzle located at a distal end of the respective extender, wherein each spray nozzle faces downward. The spraying may further comprise spraying the quench liquid downwardly from each spray nozzle as a hollow cone spray of the quench liquid, wherein each hollow cone spray defines a center.

Step 403 comprises spraying the quench liquid in a first projected spray. Step 403 may comprise spraying the quench liquid from the nozzles of the first spray bar so that the hollow cone spray of each nozzle of the first spray bar overlaps with at least one hollow cone spray of an adjacent nozzle of the first spray bar, wherein the nozzles of the first spray bar are configured to provide a plurality of first overlaps of spray, wherein the hollow cone sprays and plurality of first overlaps form a first projected spray of the quench liquid, the first projected spray of the quench liquid defining gaps between the hollow cone sprays and first overlaps.

Step 404 comprises spraying the quench liquid in a second projected spray. Step 404 may comprise spraying the quench liquid from the nozzles of the second spray bar so that the first projected spray and the second projected spray, in combination, provide a downward wall of projected spray that covers more surface area of a cross section of the quench vessel parallel to the diameter of the quench vessel than a surface area covered by the first projected spray alone.

While in the foregoing specification this disclosure has been described in relation to certain preferred embodiments thereof, and many details have been set forth for purpose of illustration, it will be apparent to those skilled in the art that the disclosure is susceptible to additional embodiments and that certain of the details described herein can be varied considerably without departing from the basic principles of the disclosure. For example, the dimensions, number, size and shape of the various components may be altered to fit specific applications. Accordingly, the specific embodiments illustrated and described herein are for illustrative purposes only.

## Claims

1. A quench vessel (10) comprising;
an inlet (32) configured to receive a gaseous stream that includes acrylonitrile and ammonia; and
a multi-level spray system (34) configured to receive a quench liquid, the inlet located (32) below the multi-level spray system (34),
wherein the multi-level spray system (34) includes at least a first and a second spray bar (54, 56) that extends substantially across a diameter (62) of the quench vessel (10), each spray bar comprising a plurality of nozzles configured to provide a spray of quench liquid, the spray nozzles effective for providing a collective spray having a spray pattern that covers a surface area of a cross section of the quench vessel (10),
and wherein the nozzles of the first spray bar (54) are spaced so that a portion of each hollow cone spray of quench liquid from each nozzle of the first spray bar (54) overlaps with a portion of another hollow cone spray of quench liquid from at least one adjacent nozzle to provide a plurality of first overlaps of the quench liquid, wherein each first overlap has a first overlap center,
and the nozzles of the second spray bar (56) are spaced so that a portion of each hollow cone spray of quench liquid from each nozzle of the second spray bar (56) overlaps with a portion of another hollow cone spray of quench liquid from at least one adjacent nozzle to provide a plurality of second overlaps of the quench liquid, wherein each second overlap has a second overlap center,
and further wherein the nozzles of the second spray bar (56) are spaced so that the center of at least one hollow cone spray of the second spray bar (56) is vertically offset from the centers of the hollow cone sprays of the first spray bar (54) and the center of at least one hollow cone spray of the second spray bar (56) is vertically aligned with at least one first overlap center of the first spray bar (54).

2. The quench vessel of claim 1, wherein the multi-level spray system (34) extends across 85% or more of the diameter (62) of the quench vessel (10).

3. The quench vessel of claim 1, wherein the first spray bar (54) is located below the second spray bar (56) and substantially parallel to the second spray bar (56).

4. The quench vessel of claim 1, wherein the spray of quench liquid is countercurrent to the gaseous stream.

5. The quench vessel of claim 1, wherein the number of spray bars are between four and ten.

6. The quench vessel of claim 1, wherein the distance between the first and second spray bars (54, 56) is configured to achieve desired quenching of reactor effluent products, and preferably wherein the distance between spray bars is 0.65 meters to 1.4 meters.

7. The quench vessel of claim 1 configured to provide a circulation rate for the quench liquid sufficient to remove the excess ammonia from the reactor effluent gas (12).

8. The quench vessel of claim 1, wherein further comprising an individual block valve and a flow meter for each spray bars, the valve and meter configured respectively to allow for adjustment and verification of flow through the corresponding spray bar.

9. The quench vessel of claim 1 configured to maintain an ammonium sulfate concentration in a stream of effluent exiting from the lower portion of the quench vessel (10) at a level from 3% to 20% by weight.

10. The quench vessel of claim 1, further comprising a stripper, the stripper configured to remove desired organics from a stream of the quenching liquid via an overhead vapor stream that is circulated back to the quench vessel and inserted back in to the vapor stream of reactor effluent beneath the sprays in the vessel.

11. The quench vessel of claim 1, wherein each spray bar includes a plurality of spray arms that extend substantially perpendicular to their respective spray bar, wherein at least one spray arm of the first spray bar and at least one spray arm of the second spray bar has two or more extenders.

12. The quench vessel of claim 11, wherein each extender extends substantially perpendicular to its respective spray arm, each extender including a spray nozzle, the spray nozzle located at a distal end its respective extender.

13. The quench vessel of claim 12, wherein the hollow cone sprays are spaced evenly throughout the vessel on a level plane such that there is approximately a 50% overlap of the projected spray pattern of the 70 to 100 degree spray outlet from the cones.

14. The quench vessel of claim 1, wherein each hollow cone spray of the first spray bar (54) has the same diameter of at least one other hollow cone spray of the first spray bar (54).

15. The quench vessel of claim 1 wherein each hollow cone spray of the second spray bar (56) has the same diameter of at least one other hollow cone spray of the second spray bar (56).

16. The quench vessel of claim 1, wherein each hollow cone spray of the first spray bar (54) has the same diameter of at least one hollow cone spray of the second spray bar (56).

17. The quench vessel of claim 12, wherein each hollow cone spray of the first spray bar (54) has the same diameter of at least one hollow cone spray of the second spray bar (56) and wherein there is at least a first pair of first and second spray bars (54, 56), and at least a second pair of a third spray bar and a fourth spray bar, wherein the third spray bar has the same configuration as the first spray bar, the fourth spray bar has the same configuration as the second spray bar, and the third spray bar is below the fourth spray bar, and the second pair of spray bars are above the first pair of spray bars.

18. The quench vessel of claim 12, wherein each nozzle is devoid of internal moving parts, and preferably wherein each hollow cone spray is configured to spray the quench liquid from the corresponding nozzle at an angle of 70 to 100 degrees.

19. The quench vessel of claim 5, comprising layers of spray bars spaced so that the center of the cones below each layer is located at the center of the overlap from the layer above, preferably wherein a two layer pattern is established, the two layer pattern configured to provide a spray of the quench liquid sufficient to provide a collective spray having coverage from the walls of the quench vessel to the center of the vessel, and most preferably wherein the two layer pattern is a first two layer pattern, and is repeated in a second two layer pattern above the first two layer pattern.

20. A process for quenching a reactor effluent comprising;
providing a gaseous stream that includes acrylonitrile and ammonia to a quench vessel (10);
providing a quench liquid to a multi-level spray system (34) in the quench vessel, wherein the quench liquid comprises sulfuric acid; and
contacting the gaseous stream with the quench liquid;
wherein the process is effective for maintaining an ammonium sulfate concentration in a stream of effluent exiting from a lower portion of the quench vessel at a concentration of 3% to 20 weight %,
wherein the multi-level spray system (34) includes at least a first and a second spray bar (54, 56) that extends substantially across a diameter (62) of the quench vessel (10), each spray bar comprising a plurality of nozzles configured to provide a spray of quench liquid countercurrent to the gaseous stream, the spray nozzles effective for providing a collective spray having a spray pattern that covers a surface area of a cross section of the quench vessel (10)
and wherein the nozzles of the first spray bar (54) are spaced so that a portion of each hollow cone spray of quench liquid from each nozzle of the first spray bar (54) overlaps with a portion of another hollow cone spray of quench liquid from at least one adjacent nozzle to provide a plurality of first overlaps of the quench liquid, wherein each first overlap has a first overlap center,
and the nozzles of the second spray bar (56) are spaced so that a portion of each hollow cone spray of quench liquid from each nozzle of the second spray bar (56) overlaps with a portion of another hollow cone spray of quench liquid from at least one adjacent nozzle to provide a plurality of second overlaps of the quench liquid, wherein each second overlap has a second overlap center,
and further wherein the nozzles of the second spray bar (56) are spaced so that the center of at least one hollow cone spray of the second spray bar (56) is vertically offset from the centers of the hollow cone sprays of the first spray bar (54) and the center of at least one hollow cone spray of the second spray bar (56) is vertically aligned with at least one first overlap center of the first spray bar (54).

21. The process of claim 20, wherein the spray of quench liquid is countercurrent to the gaseous stream.

22. The process of claim 20, wherein the multi-level spray system (34) extends across 85% or more of the diameter (62) of the quench vessel (10).

23. The process of claim 20, wherein the spray pattern covers 85% or more of the surface area of the cross section of the quench vessel (10).

24. The process of claim 20, wherein the distance between the first and second spray bars (54, 56) is configured to achieve desired quenching of reactor effluent products.

25. The process of claim 20 configured to provide a circulation rate for the quench liquid sufficient to remove at least a portion of the excess ammonia from the reactor effluent gas, and preferably wherein the circulation rate for the quench liquid is in the range of 12:1 to 25:1 based on the ratio of recycle sulfuric acid : fresh sulfuric acid, and most preferably wherein the circulation rate for the quench liquid is 18:1 based on the ratio of recycle sulfuric acid : fresh sulfuric acid.

26. The process of claim 20, wherein acid is added to the quench liquid to maintain a pH of the quench liquid of 3 to 6, and preferably wherein acid is added to the quench liquid to maintain a pH of the quench liquid of 4.5 to 6.

## Patentansprüche

1. Abschreckgefäß (10), umfassend:
einen Einlass (32), der so konfiguriert ist, dass er einen Gasstrom, der Acrylnitril und Ammoniak umfasst, aufnehmen kann; und
ein auf mehreren Ebenen befindliches Sprühsystem (34), das so konfiguriert ist, dass es eine Abschreckflüssigkeit aufnehmen kann, wobei sich der Einlass (32) unterhalb des auf mehreren Ebenen befindlichen Sprühsystems (34) befindet;
wobei das auf mehreren Ebenen befindliche Sprühsystem (34) wenigstens einen ersten und einen zweiten Sprühbalken (54, 56), der sich im Wesentlichen über einen Durchmesser (62) des Abschreckgefäßes (10) erstreckt, umfasst, wobei jeder Sprühbalken eine Vielzahl von Düsen umfasst, die so konfiguriert sind, dass sie für einen Sprühnebel aus Abschreckflüssigkeit sorgen, wobei die Sprühdüsen bewirken, dass man einen kollektiven Sprühvorgang erhält, der ein Sprühmuster aufweist, das einen Flächeninhalt eines Querschnitts des Abschreckgefäßes (10) bedeckt;
und wobei die Düsen des ersten Sprühbalkens (54) einen solchen Abstand voneinander aufweisen, dass ein Teil jedes Sprühhohlkegels aus Abschreckflüssigkeit aus jeder Düse des ersten Sprühbalkens (54) mit einem Teil eines anderen Sprühhohlkegels aus Abschreckflüssigkeit von wenigstens einer benachbarten Düse überlappt und damit für eine Vielzahl von ersten Überlappungen der Abschreckflüssigkeit sorgt, wobei jede erste Überlappung einen ersten Überlappungsmittelpunkt aufweist;
und die Düsen des zweiten Sprühbalkens (56) einen solchen Abstand voneinander aufweisen, dass ein Teil jedes Sprühhohlkegels aus Abschreckflüssigkeit aus jeder Düse des zweiten Sprühbalkens (56) mit einem Teil eines anderen Sprühhohlkegels aus Abschreckflüssigkeit von wenigstens einer benachbarten Düse überlappt und damit für eine Vielzahl von zweiten Überlappungen der Abschreckflüssigkeit sorgt, wobei jede der zweiten Überlappungen einen zweiten Überlappungsmittelpunkt aufweist;
und wobei weiterhin die Düsen des zweiten Sprühbalkens (56) einen solchen Abstand voneinander aufweisen, dass der Mittelpunkt wenigstens eines Sprühhohlkegels des zweiten Sprühbalkens (56) gegenüber den Mittelpunkten der Sprühhohlkegel des ersten Sprühbalkens (54) vertikal versetzt ist und der Mittelpunkt wenigstens eines Sprühhohlkegels des zweiten Sprühbalkens (56) vertikal an wenigstens einem ersten Überlappungsmittelpunkt des ersten Sprühbalkens (54) ausgerichtet ist.

2. Abschreckgefäß gemäß Anspruch 1, wobei sich das auf mehreren Ebenen befindliche Sprühsystem (34) über 85% oder mehr des Durchmessers (62) des Abschreckgefäßes (10) erstreckt.

3. Abschreckgefäß gemäß Anspruch 1, wobei sich der erste Sprühbalken (54) unterhalb des zweiten Sprühbalkens (56) befindet und im Wesentlichen parallel zu dem zweiten Sprühbalken (56) ausgerichtet ist.

4. Abschreckgefäß gemäß Anspruch 1, wobei sich der Sprühnebel aus Abschreckflüssigkeit im Gegenstrom zu dem Gasstrom bewegt.

5. Abschreckgefäß gemäß Anspruch 1, wobei die Anzahl der Sprühbalken zwischen vier und zehn liegt.

6. Abschreckgefäß gemäß Anspruch 1, wobei der Abstand zwischen dem ersten und dem zweiten Sprühbalken (54, 56) so konfiguriert ist, dass die gewünschte Abschreckung von Reaktorabgasprodukten erreicht wird, und wobei vorzugsweise der Abstand zwischen den Sprühbalken 0,65 Meter bis 1,4 Meter beträgt.

7. Abschreckgefäß gemäß Anspruch 1, das so konfiguriert ist, dass man eine ausreichende Zirkulationsrate für die Abschreckflüssigkeit erhält, so dass der überschüssige Ammoniak aus dem Reaktorabgas (12) entfernt wird.

8. Abschreckgefäß gemäß Anspruch 1, das weiterhin ein individuelles Blockventil und einen Durchflussmesser für jeden Sprühbalken umfasst, wobei das Ventil und das Messgerät jeweils so konfiguriert sind, dass eine Einstellung und Überprüfung des Durchflusses durch den entsprechenden Sprühbalken ermöglicht wird.

9. Abschreckgefäß gemäß Anspruch 1, das so konfiguriert ist, dass eine Ammoniumsulfatkonzentration in einem Abgasstrom, der den unteren Teil des Abschreckgefäßes (10) verlässt, auf einem Wert von 3 bis 20 Gew.-% aufrechterhalten wird.

10. Abschreckgefäß gemäß Anspruch 1, das weiterhin einen Abstreifer umfasst, wobei der Abstreifer so konfiguriert ist, dass er erwünschte organische Verbindungen über einen Kopfdampfstrom aus einem Strom von Abschreckflüssigkeit entfernt, der zurückgeführt und unterhalb der Sprühnebel in dem Gefäß wieder zurück in den Dampfstrom des Reaktorabgases eingeleitet wird.

11. Abschreckgefäß gemäß Anspruch 1, wobei jeder Sprühbalken eine Vielzahl von Sprüharmen umfasst, die sich im Wesentlichen senkrecht zu ihrem jeweiligen Sprühbalken erstrecken, wobei wenigstens ein Sprüharm des ersten Sprühbalkens und wenigstens ein Sprüharm des zweiten Sprühbalkens zwei oder mehr Verlängerungsteile aufweist.

12. Abschreckgefäß gemäß Anspruch 11, wobei sich jedes Verlängerungsteil im Wesentlichen senkrecht zu seinem jeweiligen Sprüharm erstreckt, wobei jedes Verlängerungsteil eine Sprühdüse umfasst, wobei sich die Sprühdüse an einem distalen Ende ihres jeweiligen Verlängerungsteils befindet.

13. Abschreckgefäß gemäß Anspruch 12, wobei die Sprühhohlkegel in dem gesamten Gefäß auf einer horizontalen Ebene gleichmäßig voneinander beabstandet sind, so dass es eine ungefähr 50%-ige Überlappung des ausgestoßenen Sprühmusters des 70-bis-100-Grad-Sprühaustrags aus den Kegeln gibt.

14. Abschreckgefäß gemäß Anspruch 1, wobei jeder Sprühhohlkegel des ersten Sprühbalkens (54) denselben Durchmesser wie wenigstens ein anderer Sprühhohlkegel des ersten Sprühbalkens (54) aufweist.

15. Abschreckgefäß gemäß Anspruch 1, wobei jeder Sprühhohlkegel des zweiten Sprühbalkens (56) denselben Durchmesser wie wenigstens ein anderer Sprühhohlkegel des zweiten Sprühbalkens (56) aufweist.

16. Abschreckgefäß gemäß Anspruch 1, wobei jeder Sprühhohlkegel des ersten Sprühbalkens (54) denselben Durchmesser wie wenigstens ein Sprühhohlkegel des zweiten Sprühbalkens (56) aufweist.

17. Abschreckgefäß gemäß Anspruch 12, wobei jeder Sprühhohlkegel des ersten Sprühbalkens (54) denselben Durchmesser wie wenigstens ein Sprühhohlkegel des zweiten Sprühbalkens (56) aufweist und wobei es wenigstens ein erstes Paar von ersten und zweiten Sprühbalken (54, 56) und wenigstens ein zweites Paar aus einem dritten Sprühbalken und einem vierten Sprühbalken gibt, wobei der dritte Sprühbalken dieselbe Konfiguration aufweist wie der erste Sprühbalken, der vierte Sprühbalken dieselbe Konfiguration aufweist wie der zweite Sprühbalken und sich der dritte Sprühbalken unterhalb des vierten Sprühbalkens befindet und sich das zweite Paar von Sprühbalken oberhalb des ersten Paars von Sprühbalken befindet.

18. Abschreckgefäß gemäß Anspruch 12, wobei jede Düse frei von internen beweglichen Teilen ist, wobei vorzugsweise jeder Sprühhohlkegel so konfiguriert ist, dass er die Abschreckflüssigkeit unter einem Winkel von 70 bis 100 Grad aus der entsprechenden Düse sprüht.

19. Abschreckgefäß gemäß Anspruch 5, das Schichten von Sprühbalken umfasst, die einen solchen Abstand voneinander aufweisen, dass sich der Mittelpunkt der Kegel unterhalb jeder Schicht in der Mitte der Überlappung aus der Schicht darüber befindet, wobei vorzugsweise ein zweischichtiges Muster entsteht, wobei das zweischichtige Muster so konfiguriert ist, dass es für ein ausreichendes Sprühen der Abschreckflüssigkeit sorgt, um ein kollektives Sprühen zu erhalten, das Abdeckung von den Wänden des Abschreckgefäßes bis zum Mittelpunkt des Gefäßes aufweist, wobei am meisten bevorzugt das zweischichtige Muster ein erstes zweischichtiges Muster ist, das in einem zweiten zweischichtigen Muster oberhalb des ersten zweischichtigen Musters wiederholt wird.

20. Verfahren zum Abschrecken eines Reaktorabgases, umfassend:
Leiten eines Gasstroms, der Acrylnitril und Ammoniak umfasst, in ein Abschreckgefäß (10);
Bereitstellen einer Abschreckflüssigkeit in einem auf mehreren Ebenen befindlichen Sprühsystem (34) in dem Abschreckgefäß, wobei die Abschreckflüssigkeit Schwefelsäure umfasst; und
In-Kontakt-Bringen des Gasstroms mit der Abschreckflüssigkeit;
wobei das Verfahren bewirkt, dass eine Ammoniumsulfatkonzentration in einem Abgasstrom, der den unteren Teil des Abschreckgefäßes verlässt, auf einer Konzentration von 3 bis 20 Gew.-% aufrechterhalten wird;
wobei das auf mehreren Ebenen befindliche Sprühsystem (34) wenigstens einen ersten und einen zweiten Sprühbalken (54, 56), der sich im Wesentlichen über einen Durchmesser (62) des Abschreckgefäßes (10) erstreckt, umfasst, wobei jeder Sprühbalken eine Vielzahl von Düsen umfasst, die so konfiguriert sind, dass sie für einen Sprühnebel aus Abschreckflüssigkeit sorgen, der sich im Gegenstrom zu dem Gasstrom bewegt, wobei die Sprühdüsen bewirken, dass man einen kollektiven Sprühvorgang erhält, der ein Sprühmuster aufweist, das einen Flächeninhalt eines Querschnitts des Abschreckgefäßes (10) bedeckt;
und wobei die Düsen des ersten Sprühbalkens (54) einen solchen Abstand voneinander aufweisen, dass ein Teil jedes Sprühhohlkegels aus Abschreckflüssigkeit aus jeder Düse des ersten Sprühbalkens (54) mit einem Teil eines anderen Sprühhohlkegels aus Abschreckflüssigkeit von wenigstens einer benachbarten Düse überlappt und damit für eine Vielzahl von ersten Überlappungen der Abschreckflüssigkeit sorgt, wobei jede erste Überlappung einen ersten Überlappungsmittelpunkt aufweist;
und die Düsen des zweiten Sprühbalkens (56) einen solchen Abstand voneinander aufweisen, dass ein Teil jedes Sprühhohlkegels aus Abschreckflüssigkeit aus jeder Düse des zweiten Sprühbalkens (56) mit einem Teil eines anderen Sprühhohlkegels aus Abschreckflüssigkeit von wenigstens einer benachbarten Düse überlappt und damit für eine Vielzahl von zweiten Überlappungen der Abschreckflüssigkeit sorgt, wobei jede der zweiten Überlappungen einen zweiten Überlappungsmittelpunkt aufweist;
und wobei weiterhin die Düsen des zweiten Sprühbalkens (56) einen solchen Abstand voneinander aufweisen, dass der Mittelpunkt wenigstens eines Sprühhohlkegels des zweiten Sprühbalkens (56) gegenüber den Mittelpunkten der Sprühhohlkegel des ersten Sprühbalkens (54) vertikal versetzt ist und der Mittelpunkt wenigstens eines Sprühhohlkegels des zweiten Sprühbalkens (56) vertikal an wenigstens einem ersten Überlappungsmittelpunkt des ersten Sprühbalkens (54) ausgerichtet ist.

21. Verfahren gemäß Anspruch 20, wobei sich der Sprühnebel aus Abschreckflüssigkeit im Gegenstrom zu dem Gasstrom bewegt.

22. Verfahren gemäß Anspruch 20, wobei sich das auf mehreren Ebenen befindliche Sprühsystem (34) über 85% oder mehr des Durchmessers (62) des Abschreckgefäßes (10) erstreckt.

23. Verfahren gemäß Anspruch 20, wobei das Sprühmuster 85% oder mehr des Flächeninhalts des Querschnitts des Abschreckgefäßes (10) bedeckt.

24. Verfahren gemäß Anspruch 20, wobei der Abstand zwischen dem ersten und dem zweiten Sprühbalken (54, 56) so konfiguriert ist, dass die gewünschte Abschreckung von Reaktorabgasprodukten erreicht wird.

25. Verfahren gemäß Anspruch 20, das so konfiguriert ist, dass man eine ausreichende Zirkulationsrate für die Abschreckflüssigkeit erhält, so dass wenigstens ein Teil des überschüssigen Ammoniaks aus dem Reaktorabgas entfernt wird, wobei vorzugsweise die Zirkulationsrate für die Abschreckflüssigkeit im Bereich von 12:1 bis 25:1 liegt, bezogen auf das Verhältnis von recycelter Schwefelsäure zu frischer Schwefelsäure, und wobei am meisten bevorzugt die Zirkulationsrate für die Abschreckflüssigkeit 18:1 beträgt, bezogen auf das Verhältnis von recycelter Schwefelsäure zu frischer Schwefelsäure.

26. Verfahren gemäß Anspruch 20, wobei so viel Säure zu der Abschreckflüssigkeit gegeben wird, dass ein pH-Wert der Abschreckflüssigkeit von 3 bis 6 aufrechterhalten wird, und wobei vorzugsweise so viel Säure zu der Abschreckflüssigkeit gegeben wird, dass ein pH-Wert der Abschreckflüssigkeit von 4,5 bis 6 aufrechterhalten wird.

## Revendications

1. Cuve d'extinction (10) comprenant :
une entrée (32) configurée pour recevoir un courant gazeux qui inclut de l'acrylonitrile et de l'ammoniaque ; et
un système de pulvérisation à niveaux multiples (34) configuré pour recevoir un liquide d'extinction, l'entrée disposée (32) sous le système de pulvérisation à niveaux multiples (34),
dans lequel le système de pulvérisation à niveaux multiples (34) inclut au moins une première et une seconde barre de pulvérisation (54, 56) qui s'étendent substantiellement à travers un diamètre (62) de la cuve d'extinction (10), chaque barre de pulvérisation comprenant plusieurs buses configurées pour fournir une pulvérisation de liquide d'extinction, les buses de pulvérisation efficaces pour fournir une pulvérisation collective présentant un motif de pulvérisation qui couvre une surface spécifique d'une section transversale de la cuve d'extinction (10),
et dans laquelle les buses de la première barre de pulvérisation (54) sont espacées de sorte qu'une portion de chaque pulvérisation de cône creux de liquide d'extinction à partir de chaque buse de la première barre de pulvérisation (54) chevauche une portion d'une autre pulvérisation de cône creux de liquide d'extinction à partir d'au moins une buse adjacente pour fournir plusieurs premiers chevauchements du liquide d'extinction, dans laquelle chaque premier chevauchement présente un premier centre de chevauchement,
et les buses de la seconde barre de pulvérisation (56) sont espacées de sorte qu'une portion de chaque pulvérisation de cône creux de liquide d'extinction à partir de chaque buse de la seconde barre de pulvérisation (56) chevauche une portion d'une autre pulvérisation de cône creux de liquide d'extinction à partir d'une buse adjacente pour fournir plusieurs seconds chevauchements du liquide d'extinction, dans laquelle chaque second chevauchement présente un second centre de chevauchement,
et de plus dans laquelle les buses de la seconde barre de pulvérisation (56) sont espacées de sorte que le centre d'au moins une pulvérisation de cône creux de la seconde barre de pulvérisation (56) est verticalement décalé des centres des pulvérisations de cônes creux de la première barre de pulvérisation (54) et le centre d'au moins une pulvérisation de cône creux de la seconde barre de pulvérisation (56) est verticalement aligné avec au moins un premier centre de chevauchement de la première barre de pulvérisation (54).

2. Cuve d'extinction selon la revendication 1, dans laquelle le système de pulvérisation à niveaux multiples (34) s'étend à travers 85 % ou plus du diamètre (62) de la cuve d'extinction (10).

3. Cuve d'extinction selon la revendication 1, dans laquelle la première barre de pulvérisation (54) est disposée sous la seconde barre de pulvérisation (56) et est substantiellement parallèle à la seconde barre de pulvérisation (56).

4. Cuve d'extinction selon la revendication 1, dans laquelle la pulvérisation de liquide d'extinction est à contre-courant du courant gazeux.

5. Cuve d'extinction selon la revendication 1, dans laquelle le nombre de barres de pulvérisation est de quatre à dix.

6. Cuve d'extinction selon la revendication 1, dans laquelle la distance entre les première et seconde barres de pulvérisation (54, 56) est configurée pour réaliser une extinction souhaitée de produits d'effluents de réacteur, et de préférence dans laquelle la distance entre les barres de pulvérisation est de 0,65 mètre à 1,4 mètres.

7. Cuve d'extinction selon la revendication 1, configurée pour fournir une vitesse de circulation pour le liquide d'extinction suffisante pour éliminer l'ammoniac en excès du gaz d'effluent de réacteur (12).

8. Cuve d'extinction selon la revendication 1, dans laquelle elle comprend de plus une vanne de sectionnement individuelle et un débitmètre pour chaque barre de pulvérisation, la vanne et le débitmètre configurés respectivement pour permettre un ajustement et une vérification d'écoulement à travers la barre de pulvérisation correspondante.

9. Cuve d'extinction selon la revendication 1 configurée pour maintenir une concentration en sulfate d'ammonium dans un courant d'effluent sortant de la portion inférieure de la cuve d'extinction (10) à une teneur de 3 % à 20 % en masse.

10. Cuve d'extinction selon la revendication 1, comprenant de plus un épurateur, l'épurateur configuré pour éliminer des matières organiques souhaitées d'un courant du liquide d'extinction via un courant de vapeur de tête qui est remis en circulation vers la cuve d'extinction et réinséré dans le courant de vapeur d'effluent de réacteur en dessous des pulvérisations dans la cuve.

11. Cuve d'extinction selon la revendication 1, dans laquelle chaque barre de pulvérisation inclut plusieurs bras de pulvérisation qui s'étendent substantiellement perpendiculairement à leur barre de pulvérisation respective, dans laquelle au moins un bras de pulvérisation de la première barre de pulvérisation et au moins un bras de pulvérisation de la seconde barre de pulvérisation présentent deux ou plusieurs éléments de rallonges.

12. Cuve d'extinction selon la revendication 11, dans laquelle chaque rallonge s'étend substantiellement perpendiculairement à son bras de pulvérisation respectif, chaque rallonge incluant une buse de pulvérisation, la buse de pulvérisation est disposée à une extrémité distale de sa rallonge respective.

13. Cuve d'extinction selon la revendication 12, dans laquelle les pulvérisations de cônes creux sont uniformément espacées à travers la cuve sur un plan de niveau tel qu'il y a approximativement un chevauchement de 50 % du motif de pulvérisation projetée de la sortie de pulvérisation de 70 à 100 degrés à partir des cônes.

14. Cuve d'extinction selon la revendication 1, dans laquelle chaque pulvérisation de cône creux de la première barre de pulvérisation (54) présente le même diamètre qu'au moins une autre pulvérisation de cône creux de la première barre de pulvérisation (54).

15. Cuve d'extinction selon la revendication 1, dans laquelle chaque pulvérisation de cône creux de la seconde barre de pulvérisation (56) présente le même diamètre qu'au moins une autre pulvérisation de cône creux de la seconde barre de pulvérisation (56).

16. Cuve d'extinction selon la revendication 1, dans laquelle chaque pulvérisation de cône creux de la première barre de pulvérisation (54) présente le même diamètre qu'au moins une pulvérisation de cône creux de la seconde barre de pulvérisation (56).

17. Cuve d'extinction selon la revendication 12, dans laquelle chaque pulvérisation de cône creux de la première barre de pulvérisation (54) présente le même diamètre qu'au moins une pulvérisation de cône creux de la seconde barre de pulvérisation (56) et dans laquelle il y a au moins une première paire de première et seconde barres de pulvérisation (54, 56), et au moins une seconde paire d'une troisième barre de pulvérisation et d'une quatrième barre de pulvérisation, dans laquelle la troisième barre de pulvérisation présente la même configuration que la première barre de pulvérisation, la quatrième barre de pulvérisation présente la même configuration que la seconde barre de pulvérisation, et la troisième barre de pulvérisation est en dessous de la quatrième barre de pulvérisation, et la seconde paire de barres de pulvérisation est au-dessus de la première paire de barres de pulvérisation.

18. Cuve d'extinction selon la revendication 12, dans laquelle chaque buse est dépourvue de parties mobiles internes, et de préférence dans laquelle chaque pulvérisation de cône creux est configurée pour pulvériser le liquide d'extinction à partir de la buse correspondante à un angle de 70 à 100 degrés.

19. Cuve d'extinction selon la revendication 5, comprenant des couches de barres de pulvérisation espacées de sorte que le centre des cônes sous chaque couche est disposé au centre du chevauchement à partir de la couche supérieure, de préférence dans laquelle un motif à deux couches est établi, le motif à deux couches configuré pour fournir une pulvérisation du liquide d'extinction suffisante pour fournir une pulvérisation collective présentant une couverture à partir des parois de la cuve d'extinction jusqu'au centre de la cuve, et encore mieux dans laquelle le motif à deux couches est un premier motif à deux couches et est répété dans un second motif à deux couches au-dessus du premier motif à deux couches.

20. Procédé d'extinction d'un effluent de réacteur comprenant ;
la fourniture d'un courant gazeux qui inclut de l'acrylonitrile et de l'ammoniaque dans une cuve d'extinction (10) ;
la fourniture d'un liquide d'extinction dans un système de pulvérisation à niveaux multiples (34) dans la cuve d'extinction, dans lequel le liquide d'extinction comprend de l'acide sulfurique ; et
la mise en contact du courant gazeux avec le liquide d'extinction ;
dans lequel le procédé est efficace pour maintenir une concentration en sulfate d'ammonium dans un courant d'effluent sortant d'une portion inférieure de la cuve d'extinction à une concentration de 3 % à 20 % en masse,
dans lequel le système de pulvérisation à niveaux multiples (34) inclut au moins une première et une seconde barre de pulvérisation (54, 56) qui s'étendent substantiellement à travers un diamètre (62) de la cuve d'extinction (10), chaque barre de pulvérisation comprenant plusieurs buses configurées pour fournir une pulvérisation de liquide d'extinction à contre-courant du courant gazeux, les buses de pulvérisation efficaces pour fournir une pulvérisation collective présentant un motif de pulvérisation qui couvre une surface spécifique d'une section transversale de la cuve d'extinction (10)
et dans lequel les buses de la première barre de pulvérisation (54) sont espacées de sorte qu'une portion de chaque pulvérisation de cône creux de liquide d'extinction de chaque buse de la première barre de pulvérisation (54) chevauche une portion d'une autre pulvérisation de cône creux de liquide d'extinction d'une autre buse adjacente pour fournir plusieurs premiers chevauchements du liquide d'extinction, dans lequel chaque premier chevauchement présente un premier centre de chevauchement,
et les buses de la seconde barre de pulvérisation (56) sont espacées de sorte qu'une portion de chaque pulvérisation de cône creux de liquide d'extinction de chaque buse de la seconde barre de pulvérisation (56) chevauche une portion d'une autre pulvérisation de cône creux de liquide d'extinction d'au moins une buse adjacente pour fournir plusieurs seconds chevauchements du liquide d'extinction, dans lequel chaque second chevauchement présente un second centre de chevauchement,
et de plus dans lequel les buses de la seconde barre de pulvérisation (56) sont espacées de sorte que le centre d'au moins une pulvérisation de cône creux de la seconde barre de pulvérisation (56) est verticalement décalé à partir des centres des pulvérisations de cônes creux de la première barre de pulvérisation (54) et le centre d'au moins une pulvérisation de cône creux de la seconde barre de pulvérisation (56) est verticalement aligné avec au moins un premier centre de chevauchement de la première barre de pulvérisation (54).

21. Procédé selon la revendication 20, dans lequel la pulvérisation de liquide d'extinction est à contre-courant du courant gazeux.

22. Procédé selon la revendication 20, dans lequel le système de pulvérisation à niveaux multiples (34) s'étend à travers 85 % ou plus du diamètre (62) de la cuve d'extinction (10).

23. Procédé selon la revendication 20, dans lequel le motif de pulvérisation couvre 85 % ou plus de la surface spécifique de la section transversale de la cuve d'extinction (10).

24. Procédé selon la revendication 20, dans lequel la distance entre les première et seconde barres de pulvérisation (54, 56) est configurée pour réaliser une extinction souhaitée de produits d'effluents de réacteur.

25. Procédé selon la revendication 20, configuré pour fournir une vitesse de circulation pour le liquide d'extinction suffisante pour éliminer au moins une portion de l'ammoniaque en excès du gaz d'effluent de réacteur, et de préférence dans lequel le taux de circulation pour le liquide d'extinction se trouve dans l'intervalle de 12:1 à 25:1 sur la base du rapport d'acide sulfurique recyclé : acide sulfurique frais, et encore mieux dans lequel le taux de circulation pour le liquide d'extinction est de 18:1 sur la base du taux d'acide sulfurique recyclé : acide sulfurique frais.

26. Procédé selon la revendication 20, dans lequel de l'acide est ajouté au liquide d'extinction pour maintenir un pH du liquide d'extinction à de 3 à 6, et de préférence dans lequel de l'acide est ajouté au liquide d'extinction pour maintenir un pH du liquide d'extinction à de 4,5 à 6.
